# EUROPÄISCHE PATENTSCHRIFT

(11) **EP 2 542 142 B1**
(45) Veröffentlichungstag und Bekanntmachung des Hinweises auf die Patenterteilung: **17.11.2021**
(21) Anmeldenummer: 11706599.5
(22) Anmeldetag: 03.03.2011
(51) Int. Cl.: A61B 5/00, G01N 33/493

(54) **SYSTEM ZUR VERABREICHUNG VON MEDIKAMENTEN UNTER BERÜCKSICHTIGUNG VON URINWERTEN**
SYSTEM FOR ADMINISTERING MEDICAMENTS ON THE BASIS OF URINE VALUES
SYSTÈME D'ADMINISTRATION DE MÉDICAMENTS AVEC PRISE EN COMPTE DE VALEURS URINAIRES

(30) Priorität: 24.03.2010 DE 102010012733; 05.03.2010 DE 102010010567
(43) Veröffentlichungstag der Anmeldung: 09.01.2013
(73) Patentinhaber: B. Braun Melsungen AG, 34212 Melsungen (DE)
(72) Erfinder: MAIER, Hans-Otto, 34212 Melsungen (DE); SCHMOLL, Horst, 34302 Guxhagen (DE); PAETZOLD, Matthias, 34212 Melsungen (DE)
(74) Vertreter: Winter, Brandl - Partnerschaft mbB
(86) Internationale Anmeldenummer: PCT/EP2011/053245
(87) Internationale Veröffentlichungsnummer: WO 2011/107568

(56) Entgegenhaltungen:
- DE-U1- 20 217 847
- US-A1- 2007 088 333
- US-A1- 2008 027 409
- US-A1- 2008 089 993
- US-A1- 2008 221 512
- US-A1- 2009 062 730
- US-A1- 2010 036 310

## Beschreibung

Die Erfindung betrifft ein System zur Verabreichung von Medikamenten für einen Patienten.

Patienten, die insbesondere auf der Intensivstation liegen, werden herkömmlicherweise mit Medikamenten und gegebenenfalls künstlicher Nahrung mittels einer oder mehrerer Zuführeinrichtungen, beispielsweise intravenös versorgt. Als Zuführeinrichtungen können Infusionspumpen verwendet werden, die lebenswichtige Blutwerte des Patienten auf ein vorbestimmbares Niveau in Reaktion auf eine zuvor durchgeführte Blutbild/-gasanalyse im Blutkreislauf des Patienten hält.

Sämtliche derartige Zuführeinrichtungen, auch wenn sie in ein System zur Verabreichung von Medikamenten und/oder Nahrungswerten integriert sind, erfordern bisher die durch einen Arzt oder ein weiteres klinisches Personal zu erfolgende Eingabe von Werten, die der Zuführung mittels der Zuführeinrichtung zugrunde liegen. Beispielsweise können hier Mengenwerte, Zeitabstände, in welchen die Zuführung erfolgen soll, intervallartige Zuführungen etc. als Basis für die anschließend zu erfolgende Zuführung von Medikamenten eingegeben werden.

Dieser Zuführung vorausgehend ist eine zumeist von Hand durchgeführte Blutentnahme bei dem Patienten, welche die Zwischenschaltung von klinischem Personal erfordert. Ebenso ist weiteres klinisches Personal erforderlich, welches die notwendigen Fachkenntnisse zu den Eingabefunktionen der Zuführeinrichtung, wie der Infusionspumpe hat, um anschließend diese Zuführung durchzuführen.

In der Regel sind derartige Infusionstherapien, insbesondere, wenn sie unter Einbeziehung von Urinwerten durchgeführt werden, heutzutage nicht zielwertbestimmt. Vielmehr ist die übliche Vorgehensweise die Einstellung einer Infusionsrate anhand von einem Urinwertestatus und/oder einem Blutbildwertestatus auf Basis der Erfahrung des klinischen Personals und/oder vorgegebenen Standards, Protokollen, Anweisungen, Vorgaben und dergleichen.

US 2009/0062730 A1 offenbart ein System, das die ausgeschiedene Urinmenge eines Patienten über ein Zeitintervall misst.

US 2008/0027409 A1 offenbart ein System zur Verabreichung von Flüssigkeiten an einen Patienten, bei welchem die Menge des vom Patienten ausgeschiedenen Urins gemessen wird und in Abhängigkeit dieser gemessenen Urinmenge die Zuführrate einer dem Patienten zugeführten Flüssigkeitsmenge bestimmt wird.

US 2007/088333 A1 offenbart ein System zur Verabreichung von Diuretika, bei dem aus einer Natrium-Konzentration im Urin und einer ausgeschiedenen Urinmenge die ausgeschiedene Menge an Natrium berechnet und mit einer höher konzentrierten Natriuminfusionslösung wieder zugeführt wird, um ein bestimmte Entwässerung des Körpers zu erzielen.

Ein System zur Verabreichung von Medikamenten für einen Patienten ist ferner aus US 2010/0036310 A1 bekannt

Demzufolge ist es Aufgabe der Erfindung, ein System zur Verabreichung von Medikamenten für einen Patienten zur Verfügung zu stellen, welches auch unter Berücksichtigung von Urinwerten und einer Fluidbilanzierung eine Zuführung von Medikamenten mit den richtigen Medikamentenparametern in automatisierter Weise ermöglicht.

Diese Aufgabe wird durch ein System mit den Merkmalen des Patentanspruches 1 gelöst.

Ein derartiges System ermöglicht vorteilhaft die Berücksichtigung von gemessenen Urinwerten bei der Berechnung von den Medikamentenparametern der durch die Zuführeinrichtung zu verabreichenden Medikamente. Beispielsweise kann in automatischer weise mittels der Auswerte- und der Berechnungseinrichtung der Wirkstoff Furosemid dem Patienten mittels der Zuführeinrichtung in Abhängigkeit von den zuvor gemessenen Urin- und Blutwerten verabreicht werden. Dies ermöglicht eine zusätzliche Kontrolle der gemessenen Werte für eine optimierte Verabreichung des Wirkstoffes und für eine optimierte Einstellung der Infusionsrate, wobei anstelle des Wirkstoffes Furosemid auch andere Medikamente zugeführt werden können.

Es ist zudem möglich, dass konkurrierende Werte, zum Beispiel Harnstoff, Kreatinin, Lysc, etc. aus der Urinwertemessung und aus der Blutanalyseauswertung erkannt werden und von der Berechnungseinrichtung automatisiert korrigiert werden können. Die Berechnungseinrichtung kann zudem in einer Relation zwischen einzelnen, gemessenen Werten und die zwischen diesen Werten bestehenden Differenzen bei der Berechnung eines optimierten Infusionswertes berücksichtigen und ist somit nicht nur auf die Messung absoluter Werte angewiesen. Ebenso kann die Berechnungseinrichtung bei der Berechnung der Infusionswerte, wie beispielsweise einer Infusionsrate, die Chronologie und Sequenz der zuvor gemessenen Werte dieses Patienten sowie deren Aussagequalität mitberücksichtigen.

Bei den durch die erste Messeinrichtung gemessenen Urinwerte kann es sich neben den vorgenannten Werten um weitere Urinwerte, wie beispielsweise um pH-Werte, den Eiweißgehalt, den Glukosegehalt des Urins, den Nitritgehalt des Urins, den Bilirubingehalt des Urins, den Ketonegehalt des Urins, den Bakteriengehalt des Urins und/oder Urinsedimentergebnisse handeln.

Mit einer zweiten Messeinrichtung wird die Menge des vom Patienten ausgeschiedenen Urins und eine damit verbundene zweite Auswerteeinrichtung zum Auswerten der gemessenen Urinmenge gemessen. Zudem kann durch die zweite Messeinrichtung zusätzlich die Menge des abgeschiedenen Sekrets von Wunden des Patienten gemessen werden und von der zweiten Auswerteeinrichtung entsprechend ausgewertet werden.

Dies dient dazu, dass mittels einer zweiten Berechnungseinrichtung eine Fluidbilanz des Körpers des Patienten aus der Menge der ausgeschiedenen Flüssigkeiten und der zugeführten Flüssigkeiten berechnet wird. Bei den ausgeschiedenen Flüssigkeiten handelt es sich um die zuvor ausgewertete, gemessene Urinmengeund bei den zugeführten Flüssigkeiten handelt es sich um die zugeführten flüssigen Medikamente und flüssigen Nahrungswerte. Hieraus werden zweite Medikamentenparameter berechnet.

Eine dritte Messeinrichtung dient zum Messen von Vitalwerten des Patienten, wie EKG-und EEG-Daten, und eine damit verbundene Auswerteeinrichtung dient zum Auswerten der gemessenen Vitalwerte. Mittels einer dritten Berechnungseinrichtung werden dritte Medikamentenparameter auf Basis der ausgewerteten, gemessenen Vitalwerte berechnet.

Vorteilhaft können in einer automatisierten Weise mittels einer gemeinsamen Berechnungseinrichtung aus den ersten, zweiten und dritten Medikamentenparametern die gemeinsamen Medikamentenparameter berechnet werden. Dies hat zur Folge, dass sämtliche Medikamentenparameter, die aus den berechneten Urinwerten, den berechneten Blutwerten, den Flüssigkeitsbilanzwerten und den Vitalwerten entstanden sind, für die Berechnung von optimierten, gemeinsamen Medikamentenparametern zur Einleitung der Infusion mit einer optimierten Infusionsrate berücksichtigt werden.

Vorteilhaft können mit der ersten Messeinrichtung auch die Blutwerte des Patienten gemessen werden, die von der ersten Auswerteeinrichtung auswertbar und von der ersten Berechnungseinrichtung für die Berechnung der ersten Medikamentenparameter verwendbar sind.

Es sollen somit unter Berücksichtigung einer großen Anzahl von gemessenen Werten, insbesondere unter Berücksichtigung der Urinwerte, ein automatisiertes Funktionieren des Gesamtsystems und ein dazugehöriges Verfahren zur Verabreichung von Medikamenten zur Verfügung gestellt werden, wobei die Übertragung der jeweiligen Werte, Medikamentenparameter und sonstiger Daten in automatisierter Weise erfolgen kann. Es ist auch denkbar, dass die für die beabsichtigte Infusion für eine Therapie des Patienten ermittelten Medikamentenparameter und Nahrungswerteparameter als vorgeschlagene Parameter bzw. Daten zur Unterstützung des klinischen Personals auf einer entsprechenden Anzeige an dem Patientenbett des zu behandelnden Patienten angezeigt werden, sodass das klinische Personal die Infusionspumpe mittels dieser angezeigten und ihm eingegebenen Daten in Gang setzt.

Um eine sichere Übertragung in automatisierter Weise von den einzelnen Messeinrichtungen, Auswerteeinrichtungen und Berechnungseinrichtungen untereinander und zu dem Infusionssystem, das beispielsweise eine oder mehrere Infusionspumpen sein kann, sicherzustellen, werden diese Daten mittels eines Datenprotokolls, welches eine Patienten-ID, den Zeitpunkt der stattgefundenen Messung, den Zeitpunkt der Probenentnahme, die Identifikation der jeweiligen Messeinrichtung, die Qualitätsklasse der Messeinrichtung sowie weitere Qualitätsmerkmale enthalten, übertragen.

Die Flüssigkeitsbilanzierung findet in automatisierter Weise statt und die Ermittlung ausgeschiedener Flüssigkeiten wird nicht mittels komplizierter Wägetechniken durchgeführt. Vielmehr sollen sämtliche ausgeschiedenen messbaren Flüssigkeiten vorzugsweise in automatisierter Form in ihrer Menge ermittelt werden und die restlichen Flüssigkeiten, welche ein menschlicher Körper verliert, durch Berechnung simuliert werden. Diesen ausgeschiedenen Flüssigkeiten werden die zugeführten Flüssigkeiten in Form von flüssigen Medikamenten oder Nährstoffen, welche sich einfach ermitteln lassen, gegenübergestellt.

Weitere vorteilhafte Ausführungsformen ergeben sich aus den Unteransprüchen.

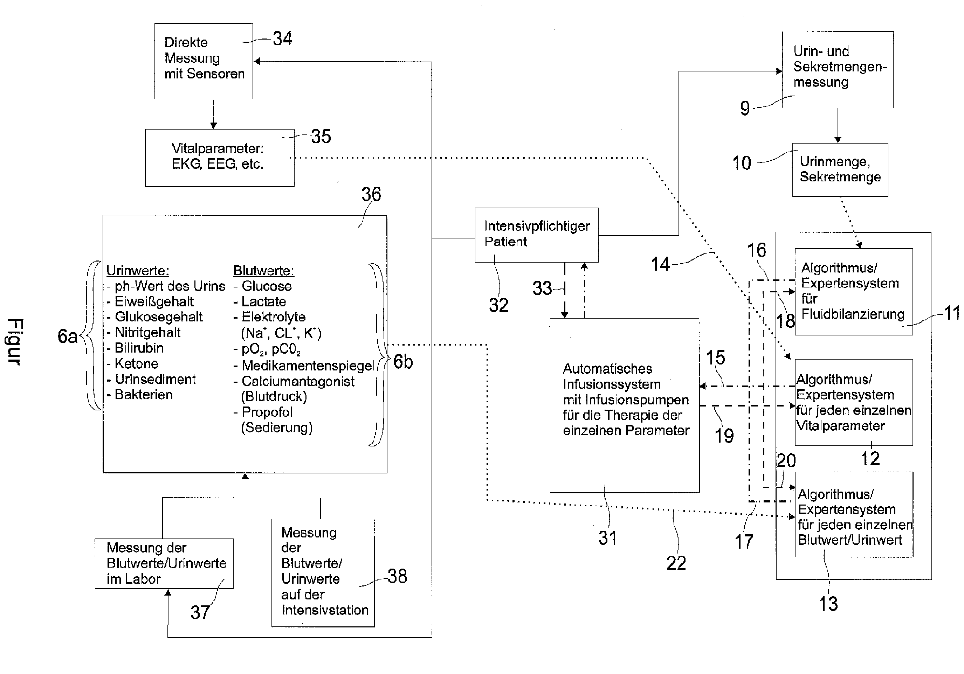

Vorteile und Zweckmäßigkeiten sind der nachfolgenden Beschreibung in Verbindung mit der Zeichnung zu entnehmen.

Es zeigt die einzige Figur eine schematische Darstellung des Systems zur Verabreichung von Medikamenten gemäß einer Ausführungsform der Erfindung.

Bei dem in dieser Figur dargestellten System für die automatisierte Verabreichung von Medikamenten (Beispiele sind Wirkstoffe (Arzneimittel) wie Furosemid, Insulin etc) ist ein automatisches Infusionssystem 1 mit einer Mehrzahl von Infusionspumpen für die Therapie der einzelnen falsch eingestellten Werte innerhalb des Körpers des Patienten 2 gegeben.

Ein intensivpflichtiger Patient 2 erhält somit gemäß dem Bezugszeichen 3 von dem automatisierten Infusionssystem 1 Medikamente mit den dazugehörigen Infusionsraten, wobei gegebenenfalls eine Veränderung der Medikamentenmenge, des Verabreichungszeitraums und weiterer Parameter berücksichtigt wird.

Der intensivpflichtige Patient 2 kann mittels einer oder mehrerer Messeinrichtungen 4, 7, 8 oder 9 verschiedenartig, vorrangig in seinen Urinwerten, analysiert werden.

Beispielsweise können mittels der Messeinrichtungen 8 in automatisierter Weise auf der Intensivstation die Urinwerte und/oder die Blutwerte durch Entnahme von Urin und Blut von Patienten 2 gemessen werden. Im Einzelnen handelt es sich gemäß Bezugszeichen 6a bei den Urinwerten um den pH-Wert des Urins, den Eiweißgehalt des Urins, den Glukosegehalt des Urins, den Nitritgehalt des Urins, den Bilirubingehalt des Urins, den Ketonegehalt des Urins, den Bakteriengehalt des Urins und Urinsedimentergebnissen. Bei den Blutwerten handelt es sich im Einzelnen um den Glukosewert, den Laktatwert den Elektrolytewert (Na⁺, CL⁺, K⁺), den pO₂-Wert, den PCO₂-Wert, Medikamentenspiegelwerten, Kalziumantagonistwerten (Blutdruck) und den Propofolwerten (Sedierung).

Die Blutwerte werden gemäß dem Bezugszeichen 6b wiedergegeben. Zusätzlich können mittels einer weiteren Messeinrichtung 9 die Urin- und Sekretmengenwerte vorzugsweise in automatisierter Weise gemessen werden. Daraus werden mittels einer Auswerteeinrichtung 10 die Urinmengen- und Sekretmengenwerte ermittelt.

Mittels einer Messeinrichtung 4 werden vorzugsweise mittels Sensoren in automatisierter Weise Vitalparameter wie EKG- und EEG-Werte ermittelt, welche in einer dritten Auswerteeinrichtung 5 ausgewertet werden.

Von sämtlichen Auswerteeinrichtungen 5, 6 und 10 werden die ausgewerteten Werte in vorzugsweise automatisierter Form an eine jeweils dazugehörige Berechnungseinrichtung 11, 12 und 13 übertragen. Die Übertragung findet gemäß dem Bezugszeichen 14, 21 und 22 mittels eines Datenprotokolls statt, welches zusätzlich Daten aufweist, wie die Patienten-Identifikationsnummern, den Zeitpunkt der stattgefundenen Messung, den Zeitpunkt der Probenentnahme, die Identifikation der jeweiligen Messeinrichtung, die Qualitätsklasse der Messeinrichtung sowie ein zugeordnetes Qualitätsmerkmal.

In der Berechnungseinrichtung 11 wird eine Berechnung der relevanten Medikamentenparameter und Nahrungswertparameter für die Verabreichung von Medikamenten und Nahrungswerten mit dem Infusionssystem 1 berechnet. Hierfür wird für eine durchzuführende Flüssigbilanzierung eine Bilanzierung sämtlicher ausgeschiedener Flüssigkeiten, also die Urinmenge und zusätzlich beispielsweise eine gemessene Sekretmenge, und der zugeführten Flüssigkeiten, wie beispielsweise die flüssigen Medikamente und flüssigen Nahrungswerte gemäß dem Infusionssystem 1 durchgeführt.

Zu einer derartigen Flüssigkeitsbilanzierung aus infundierten Flüssigkeiten versus ausgeschiedener Flüssigkeiten ist Folgendes zu erwähnen:
Menschliche Wesen scheiden bekannterweise Flüssigkeiten auf unterschiedlichster Weise aus. Zum Einen werden Urinmengen und Wundsekretmengen ausgeschieden, die bei intensivpflichtigen Patienten automatisiert und direkt gemessen werden können. Zu den unmittelbaren messbaren Flüssigkeiten gibt es noch mittelbar feststellbare Flüssigkeiten, wie ausgeschiedener Wasserdampf durch Atmung, die Transpiration durch die Haut des Körpers und die Flüssigkeitsausscheidung durch Stuhl und dergleichen. Diese Flüssigkeitsmengen werden mittels physiologischer Modelle unter Berücksichtigung der Körperoberfläche, der Körpertemperatur und dergleichen errechnet.

Mit der Berechnungseinrichtung 12 lassen sich die zweiten Medikamentenparameter für die zu verabreichenden Medikamente mittels des Infusionssystems 1 und gegebenenfalls dazugehörigen Nahrungswerte berechnen.

Mittels der Berechnungseinrichtung 13 lassen sich auf Grundlage der gemessenen Urinwerte und/oder Blutwerte die Medikamentenparameter und Nahrungswerteparameter für die zu verabreichenden Medikamente und Nahrungswerte mittels des Infusionssystems 1 berechnen.

Die berechneten Medikamentenparameter und Nahrungswerteparameter werden gemäß den Bezugszeichen 15, 16 und 17 an das Infusionssystem 1 drahtgebunden oder drahtlos, vorzugsweise verschlüsselt, übertragen.

Bei einer Veränderung der Infusionsraten der zu verabreichenden Infusionen bzw. Medikamente und Nahrungswerte findet eine Rückkopplung an die Berechnungseinrichtung 11, 12 und 13 gemäß den Bezugszeichen 18, 19 und 20 statt, sodass diese Veränderungen bei der Neuberechnung von Medikamentenparametern und/oder Nahrungswerteparametern berücksichtigt werden können.

Sämtliche Berechnungseinrichtungen 11, 12 und 13 sind innerhalb einer gemeinsamen Berechungseinrichtung zusammengefasst.

Im Übrigen können die Urinwerte und Blutwerte ebenso mittels einer Messeinrichtung 7 innerhalb eines Labors ermittelt werden.

Sofern von den verschiedenen Messeinrichtungen sich konkurrierende Werte gemessen werden, können die Berechnungseinrichtungen 11, 12 und 13 dies berücksichtigen und eine entsprechende Korrektur unter Berücksichtigung der Wertigkeit den einzelnen Messeinrichtungen und der damit gemessenen Werte durchführen.

Ebenso ist es denkbar, dass die Berechnungseinrichtungen die Chronologie der Messungen, diese Sequenz der Messungen sowie die Qualität der Messungen bei der Ermittelung der Medikamentenparameter und/oder der Nahrungswerteparameter mitberücksichtigen. Auch werden Relationen und Differenzen der einzelnen ermittelten Werte in Bezug zueinander und in Bezug zu anderen Werten von anderen Messeinrichtungen bei der Ermittlung der Medikamentenparameter und/oder der Nahrungswerteparameter berücksichtigt.

### Bezugszeichenliste

- 1: Infusionssystem
- 2: Patient
- 3: Medikamente
- 4: dritte Messeinrichtung
- 5: dritte Auswerteeinrichtung
- 6: erste Auswerteeinrichtung
- 6a: Urinwerte
- 6b: Blutwerte
- 7: erste Messeinrichtung im Labor
- 8: erste Messeinrichtung auf der Intensivstation
- 9: zweite Messeinrichtung
- 10: zweite Auswerteeinrichtung
- 11: erste Berechnungseinrichtung
- 12: zweite Berechnungseinrichtung
- 13: dritte Berechnungseinrichtung
- 14: Übertragung der Werte mittels Datenprotokoll
- 15: Übertragung der Medikamenten- und Nahrungsparameter an das Infusionssystem
- 16: Übertragung der Medikamenten- und Nahrungsparameter an das Infusionssystem
- 17: Übertragung der Medikamenten- und Nahrungsparameter an das Infusionssystem
- 18: Rückkopplung an die Berechnungseinrichtung
- 19: Rückkopplung an die Berechnungseinrichtung
- 20: Rückkopplung an die Berechnungseinrichtung
- 21: Übertragung der Werte mittels Datenprotokoll
- 22: Übertragung der Werte mittels Datenprotokoll

## Patentansprüche

1. System zur Verabreichung von Medikamenten für einen Patienten (2) umfassend:
- mindestens eine Zuführeinrichtung (1) zum Zuführen von flüssigen Medikamenten und flüssigen Nahrungswerten;
- eine erste Messeinrichtung (7, 8) zum Messen von Urinwerten (6a) des Urins eines Patienten (2);
- eine erste Auswerteeinrichtung (6) zum Auswerten der gemessenen Urinwerte (6a); und
- eine erste Berechnungseinrichtung (13) zum Berechnen von ersten Medikamentenparametern der den jeweiligen Patienten (2) zu verabreichenden Medikamente auf Basis der ausgewerteten, gemessenen Urinwerte (6a);
- eine zweite Messeinrichtung (9) zum Messen der Menge des vom Patienten (2) ausgeschiedenen Urins;
- eine zweite Auswerteeinrichtung (10) zum Auswerten der gemessenen Urinmenge; und
- eine zweite Berechnungseinrichtung (11) zum Berechnen einer Fluidbilanz des Körpers des Patienten (2) aus der ausgeschiedenen Urinmenge und aus einer durch die mindestens eine Zuführeinrichtung (1) zugeführten Menge an flüssigen Medikamenten und flüssigen Nahrungswerten, und zum Berechnen zweiter Medikamentenparameter aus der berechneten Fluidbilanz; wobei
- die mindestens eine Zuführeinrichtung (1) die flüssigen Medikamente mit einer auf der Grundlage von zumindest aus den ersten und zweiten Medikamentenparametern berechneten, gemeinsamen Medikamentenparametern eingestellten Infusionsrate zuführt.

2. System nach Anspruch 1, **dadurch gekennzeichnet, dass**
mit der ersten Messeinrichtung (7, 8) als Urinwerte (6a) der pH-Wert, der Eiweißgehalt, der Glukosegehalt des Urins, der Nitratgehalt des Urins, der Bilirubingehalt des Urins, der Ketonegehalt des Urins, der Bakteriengehalt des Urins und/oder Urinsedimentergebnisse messbar sind.

3. System nach Anspruch 1, **dadurch gekennzeichnet, dass**
durch die zweite Messeinrichtung (9) zusätzlich die Menge des abgeschiedenen Sekrets von Wunden des Patienten (2) messbar und von der zweiten Auswerteeinrichtung (10) auswertbar sind.

4. System nach Anspruch 3, **dadurch gekennzeichnet, dass**
die zweite Berechnungseinrichtung (11) die Fluidbilanz des Körpers des Patienten (2) aus der Menge der ausgeschiedenen Urinmenge und der ausgeschiedenen Sekretmenge, und aus der durch die mindestens eine Zuführeinrichtung (1) zugeführten Menge an flüssigen Medikamenten und flüssigen Nahrungswerte berechnet und hieraus die zweiten Medikamentenparameter berechnet.

5. System nach einem der vorangegangenen Ansprüche, **gekennzeichnet durch** eine dritte Messeinrichtung (4) zum Messen von Vitalwerten des Patienten (2) und eine damit verbundene dritte Auswerteeinrichtung (5) zum Auswerten der gemessenen Vitalwerte.

6. System nach Anspruch 5, **gekennzeichnet durch**
eine dritte Berechnungseinrichtung (12) zum Berechnen dritter Medikamentenparameter auf Basis der ausgewerteten gemessenen Vitalwerte.

7. System nach Anspruch 6, **gekennzeichnet durch**
eine gemeinsame Berechnungseinrichtung, welche aus den ersten, zweiten und dritten Medikamentenparametern die gemeinsamen Medikamentenparameter berechnet.

8. System nach einem der vorangegangenen Ansprüche, **dadurch gekennzeichnet, dass**
mit der ersten Messeinrichtung (7, 8) auch Blutwerte (6b) des Patienten (2) messbar sind, die von der ersten Auswerteeinrichtung (6) auswertbar und von der ersten Berechnungseinrichtung (13) für die Berechnung der ersten Medikamentenparameter verwendbar sind.

## Claims

1. System for administration of medicaments for a patient (2) comprising:
- at least one supply device (1) to supply liquid medicaments and liquid nutrients;
- a first measurement device (7, 8) to measure urine values (6a) of the urine of a patient (2);
- a first analysis device (6) to analyse the measured urine values (6a); and
- a first calculation device (13) to calculate first medicament parameters of the medicaments to be administered to the respective patient (2) on the basis of the analysed, measured urine values (6a);
- a second measurement device (9) to measure the quantity of urine eliminated by the patient (2);
- a second analysis device (10) to analyse the urine quantity measured; and
- a second calculation device (11) to calculate a fluid balance of the body of the patient (2) from the quantity of urine eliminated and from a quantity of the liquid medicaments and liquid nutrients supplied by the at least one supply device (1), and to calculate second medicament parameters from the calculated fluid balance; wherein
- the at least one supply device (1) supplies the liquid medicaments with an infusion rate set on the basis of common medicament parameters calculated on the basis of at least the first medicament parameters and the second medicament parameters.

2. System according to claim 1, **characterised in that** as urine values (6a), the pH value, the protein content, the glucose content of the urine, the nitrate content of the urine, the bilirubin content of the urine, the ketone content of the urine, the bacteria content of the urine and/or the urine sediment results can be measured with the first measurement device (7, 8).

3. System according to claim 1, **characterised in that** also the quantity of secretion eliminated from the wounds of the patient (2) can be measured with the second measurement device (9) and can be analysed by the second analysis device (10).

4. System according to claim 3, **characterised in that** the second calculation device (11) calculates the fluid balance of the body of the patient (2) from the quantity of the eliminated urine quantity and the eliminated secretion quantity, and from the quantity of the supplied liquid medicaments and liquid nutrients supplied by the at least one supply device (1), and calculates the second medicament parameters based on this.

5. System according to one of the preceding claims, **characterised by** a third measurement device (4) for measuring vital values of the patient (2), and a third analysis device (5) connected thereto for analysing the vital values measured.

6. System according to claim 5, **characterised by** a third calculation device (12) to calculate third medicament parameters on the basis of the analysed, measured vital values.

7. System according to claim 6, **characterised by** a common calculation device which calculates the common medicament parameters from the first, second and third medicament parameters.

8. System according to one of the preceding claims, **characterised in that** also blood values (6b) of the patient (2) can be measured with the first measurement device (7, 8) and can be analysed by the first analysis device (6) and can be used by the first calculation device (13) to calculate the first medicament parameters.

## Revendications

1. Système d'administration de médicaments à un patient (2) comprenant :
- au moins un dispositif de distribution (1) pour distribuer des médicaments liquides et des éléments nutritifs liquides ;
- un premier dispositif de mesure (7, 8) pour mesurer des valeurs urinaires (6a) de l'urine d'un patient (2) ;
- un premier dispositif d'évaluation (6) pour évaluer les valeurs urinaires mesurées (6a) ; et
- un premier dispositif de calcul (13) pour calculer des premiers paramètres de médicament des médicaments à administrer aux patients respectifs (2) sur la base des valeurs urinaires mesurées et évaluées (6a) ;
- un deuxième dispositif de mesure (9) pour mesurer la quantité d'urine éliminée par le patient (2) ;
- un deuxième dispositif d'évaluation (10) pour évaluer la quantité d'urine mesurée ; et
- un deuxième dispositif de calcul (11) pour calculer un équilibre des fluides du corps du patient (2) à partir de la quantité d'urine éliminée et à partir d'une quantité de médicaments liquides et d'éléments nutritifs liquides distribués par le au moins un dispositif de distribution (1), et pour calculer des deuxièmes paramètres de médicament à partir de l'équilibre des fluides calculé ; dans lequel
- le au moins un dispositif de distribution (1) distribue les médicaments liquides à un débit de perfusion réglé sur la base de paramètres de médicament communs calculés au moins à partir des premiers et deuxièmes paramètres de médicament.

2. Système selon la revendication 1, **caractérisé en ce que**
la valeur de pH, la teneur en protéines, la teneur en glucose de l'urine, la teneur en nitrate de l'urine, la teneur en bilirubine de l'urine, la teneur en cétone de l'urine, la teneur en bactéries de l'urine et/ou des résultats de sédiment urinaire peuvent être mesurés à l'aide du premier dispositif de mesure (7, 8) en tant que valeurs urinaires (6a).

3. Système selon la revendication 1, **caractérisé en ce que**
les quantités des sécrétions provenant des plaies du patient (2) peuvent en outre être mesurées par le deuxième dispositif de mesure (9) et évaluées par le deuxième dispositif d'évaluation (10).

4. Système selon la revendication 3, **caractérisé en ce que**
le deuxième dispositif de calcul (11) calcule l'équilibre des fluides du corps du patient (2) à partir de la quantité d'urine éliminée et de la quantité de sécrétions éliminées, et à partir de la quantité de médicaments liquides et d'éléments nutritifs liquides distribués par le au moins un dispositif de distribution (1), et calcule les deuxièmes paramètres de médicament à partir de celui-ci.

5. Système selon l'une quelconque des revendications précédentes, **caractérisé par**
un troisième dispositif de mesure (4) pour mesurer des fonctions vitales du patient (2) et un troisième dispositif d'évaluation (5) connecté à celui-ci pour évaluer les fonctions vitales mesurées.

6. Système selon la revendication 5, **caractérisé par**
un troisième dispositif de calcul (12) pour calculer des troisièmes paramètres de médicament sur la base des fonctions vitales mesurées évaluées.

7. Système selon la revendication 6, **caractérisé par**
un dispositif de calcul commun qui calcule les paramètres de médicament communs à partir des premiers, deuxièmes et troisièmes paramètres de médicament.

8. Procédé selon l'une quelconque des revendications précédentes, **caractérisé en ce que**
des niveaux sanguins (6b) du patient (2) peuvent également être mesurés au moyen du premier dispositif de mesure (7, 8), qui peuvent être évalués par le premier dispositif d'évaluation (6) et utilisés par le premier dispositif de calcul (13) pour le calcul des premiers paramètres de médicament.
